# EUROPÄISCHE PATENTSCHRIFT

(11) **EP 1 754 459 B1**
(45) Veröffentlichungstag und Bekanntmachung des Hinweises auf die Patenterteilung: **28.05.2008**
(21) Anmeldenummer: 06016655.0
(22) Anmeldetag: 09.08.2006
(51) Int. Cl.: A61F 5/11

(54) **Nagelkorrekturspange sowie Werkzeug zur Anbringung einer solchen Spange an einem Nagel, insbesondere einem Fussnagel**
Nail corrective brace, and tool for applying such a brace to the nail, in particular a toe nail
Dispositif corrective de ongle, et outil pour appliquer un tel dispositif à l'ongle, en particulier un ongle d'orteil

(30) Priorität: 17.08.2005 DE 102005039147
(43) Veröffentlichungstag der Anmeldung: 21.02.2007
(73) Patentinhaber: RATHENOW, Brigitte, 40181 Bedburg (DE)
(72) Erfinder: RATHENOW, Brigitte, 40181 Bedburg (DE)
(74) Vertreter: Paul, Dieter-Alfred

(56) Entgegenhaltungen:
- DE-A1- 3 711 755
- DE-U1- 20 106 072
- US-A- 5 012 799

## Beschreibung

Die vorliegende Erfindung betrifft eine Nagelkorrekturspange, insbesondere für Fußnägel, die quer über einen zu behandelnden Nagel legbar ist und endseitig jeweils einen Endhaken zur Anbringen an dem Nagel aufweist, der um eine seitliche Nagelkante legbar ist, so daß er den Nagel untergreift. Desweiteren betrifft die Erfindung ein werkzeug zur Anbringung einer solchen Nagelkorrekturspange an einem Nagel.

Im Rahmen der fußpflegerischen Tätigkeit müssen häufig insbesondere Fußnägel behandelt werden, die eine zu starke Krümmung aufweisen und daher mit ihren Außenrändern schmerzhaft ins Fleisch drücken oder sogar einwachsen können. Eine bekannte Behandlungsmethode sieht das Entfernen des Nagels oder eines Nagelteils vor. Die Folgen sind erhebliche Schmerzen bei und nach der Operation und eine längere Genesungszeit und häufige Rezidive.

Eine nicht-chirurgische Behandlungsmethode bietet die Orthonyxie, d.h. die Geradrichtung von Nägeln mittels Spangen, die mit seitlichen Endhaken unter die Nagelränder bzw. Nagelkanten greifen und diese durch Federwirkung nach oben ziehen. Hierdurch wird die Krümmung des Nagels reduziert und eine Abheilung der ggf. entzündeten Partien ermöglicht.

Aus der DE 201 06 072 U1 ist bereits eine Nagelkorrekturspange der eingangs genannten Art bekannt, die zwei Spangenteile aus Draht umfaßt, welche an ihrem einen Ende mit einem unter den Nagel greifenden Endhaken versehen sind und an ihren zueinander weisenden Enden Verbindungsösen bzw. verbindungsausbiegungen aufweisen, über welche sie mittels einer Drahtschlaufe verbunden werden können. Zur Gewährleistung einer gewissen Zugspannung auf den Nagelrand weisen die Spangenteile jeweils eine Federausbiegung auf, welche eine V-, U- oder Ω-Grundform besitzen.

Die bekannten Nagelkorrekturspangen haben sich zwar in der Praxis bewährt. Die Bestrebungen gehen jedoch dahin, die Spangen und deren Handhabung weiter zu vereinfachen und insbesondere die Präzision der erzielbaren Korrekturen zu erhöhen.

Die Aufgabe ist erfindungsgemäß dadurch gelöst, daß die Nagelkorrekturspange aus zwei drahtförmigen Spangenteilen besteht, die jeweils an ihrem einen Ende einen der Endhaken zur Anbringung an dem Nagel und beabstandet von dem Endhaken eine Verbindungsausbiegung aufweisen, um die beiden Spangenteile unmittelbar miteinander zu verbinden, und daß sich an die Verbindungsausbiegung von einem der Spangenteile ein Führungsabschnitt für die Verbindungsausbiegung des anderen Spangenteils anschließt.

Außerdem wird nach Anspruch 7 ein Werkzeug zur Anbringung der Nagelkorrekturspange vorgesehen. Erfindungsgemäß werden somit zwei drahtförmige Spangenteile zur Verfügung gestellt, die an ihrem einen Endbereich jeweils einen Endhaken und beabstandet von dem Endhaken verbindungsausbiegungen aufweisen. Das erste Spangenteil wird an einer seitlichen Nagelkante angebracht, indem der Endhaken des Spangenteils um die Nagelkante gelegt wird, so daß er den Nagel untergreift, und das zweite Spangenteil wird in gleicher Weise an der gegenüberliegenden seitlichen Nagelkante befestigt. Anschließend werden die beiden Spangenteile unmittelbar miteinander verbunden, indem Verbindungsausbiegungen der beiden Spangenteile in Eingriff miteinander gebracht werden. Dadurch, daß die erfindungsgemäße Nagelkorrekturspange nur zwei Teile benötigt, ist sie einfach im Aufbau und leicht in der Anfertigung. Desweiteren ermöglicht die erfindungsgemäße Nagelkorrekturspange eine schonende Anbringung, da die beiden Spangenteile einzeln um die seitlichen Nagelkanten herum eingelegt werden und insbesondere nicht eingeschoben, sondern punktgenau eingesetzt werden. Die zur Verbindung notwendige leichte Zugkraft kommt nach der Anbringung der Nagelkorrekturspange unmittelbar als Druckentlastung an den Nagelfalzen zum Tragen.

Dabei ist es möglich, die Verbindungsausbiegung des zweiten Spangenteils nach dessen Anbringung an dem Nagel mit dem Führungsabschnitt in Eingriff zu bringen und entlang des Führungsabschnitts in Eingriff mit der Verbindungsausbiegung des ersten Spangenteils zu ziehen. Diese Ausbildung hat somit den Vorteil, daß die Verbindung zwischen dem Führungsabschnitt des ersten Spangenteils und dem Verbindungsabschnitt des zweiten Spangenteils erfolgen kann, ohne daß die Spangenteile durch zugkräfte beansprucht sind, und die endgültige Verbindung durch Einhaken der Verbindungsausbiegungen in einfacher weise hergestellt wird, indem die Verbindungsausbiegung entlang des Führungsabschnitts in die Verbindungsausbiegung des ersten Spangenteils gezogen wird. Um diesen Vorgang zu vereinfachen, schließt sich an den Führungsabschnitt des ersten Spangenteils zweckmäßigerweise ein aufrecht stehender Drahtabschnitt an, an dem ein Griffelement vorgesehen ist, über welches das erste Spangenteil festgehalten werden kann. Außerdem ist es möglich, als weitere Einziehhilfe eine an einem Halteelement wie beispielsweise einem Griff oder einer Fingerschlaufe befestigte Schlaufe aus einem festen, aber elastischen Material wie beispielsweise Draht zu verwenden, welche in die Verbindungsausbiegung des zweiten Spangenteils eingehängt wird, um diese in den Eingriff mit der verbindungsausbiegung des ersten Spangenteils zu ziehen.

In der Regel kann es ausreichend sein, eine erfindungsgemäße Nagelkorrekturspange an einem Nagel anzubringen. Alternativ ist es auch möglich, mehrere Nagelkorrekturspangen parallel an einem Nagel anzubringen und auf diese Weise den Nagel über seine ganze Länge zu korrigieren.

Dabei werden die beiden Spangenteile während der Anbringung an dem Nagel in ihrer Länge und auch ihrer Form an den Nagel angepaßt. Beispielsweise ist es möglich, die Endhaken erst unmittelbar vor der Anbringung an dem Nagel zu fertigen, nachdem das drahtförmige Spangenteil auf eine entsprechende Länge zugeschnitten wurde, wobei auch die Form des Endhakens an die Dicke des Nagels angepaßt werden kann.

In an sich bekannter Weise können die Spangenteile eine etwa Ω- oder U-förmige Federausbiegung aufweisen, die nach der Anbringung an einem Nagel flach auf diesem aufliegt und deren offene Seite zu der freien, vorderen Nagelkante weist. Gemäß einer bevorzugten Ausführungsform ist jedoch vorgesehen, daß nur ein Spangenteil eine solche Federausbiegung aufweist. Die Anpassung des Spangenteils an den Nagel kann dann in der Weise erfolgen, daß die Ω- oder U-förmige Federausbiegung des Spangenteils auf dem höchsten Punkt des Nagels flach zu liegen kommt. Hierdurch wird sichergestellt, daß die von der Federausbiegung ausgeübten Zugkräfte gleichmäßig zu beiden Seiten wirken. Wenn der Nagel sich an einer Nagelseite stärker in den Nagelfalz eingedrückt hat und entsprechend dort eine stärkere Korrektur erforderlich ist als auf der gegenüberliegenden Nagelseite, wird der Ort der Federausbiegung zu der stärker zu korrigierenden Seite hin verlagert. Durch diese Verlagerung der Federausbiegung ist es möglich, die erzielbaren Hebelkräfte individuell auf beiden Seiten einzustellen.

Gemäß einer Ausführungsform wird das erste Spangenteil aktiviert, indem der Schenkel des Spangenteils flachgebogen wird, so daß die Federausbiegung und die Verbindungsausbiegung bei eingehängtem Spangenteil von dem Nagel hochstehen. Das Maß, um welches das Spangenteil hochgebogen wird, entspricht dabei etwa der Nageldicke, d.h. ca. 1 bis 3 mm. Auf diese Weise ist die paßgenauigkeit der Spangenform sowie die Stärke der dabei gegebenen Hebelwirkung einfach und direkt optisch bzw. per Fingerdruck zu kontrollieren.

In weiterer Ausbildung dieser Ausführungsform ist vorgesehen, daß die Federausbiegung unmittelbar neben der Verbindungsausbiegung des ersten Spangenteils vorgesehen ist, wobei die Verbindungsausbiegung ebenfalls Ω- oder U-förmig ausgebildet sein kann und so ausgerichtet ist, daß ihr offenes Ende von der freien, vorderen Nagelkante wegweist. Dabei ist die Verbindungsausbiegung quer zu der Ebene der Federausbiegung ausgerichtet, so daß die Verbindungsausbiegung bei an einem Nagel befestigter Spange von dem Nagel nach oben absteht und die Verbindungsausbiegung des anderen Spangenteils eingehakt werden kann.

Zur Festestellung des Erfolges der Nagelkorrektur kann ein Prüfmittel eingesetzt werden. Hierzu wird die Skala parallel zu der vorderen, freien Nagelkante angelegt, um die Breite des gerade auszurichtenden Nagels abzulesen.

Zur optimalen Ablesbarkeit kann die Skala unterschiedlich eingefärbt sein. Beispielsweise ist es möglich, im Wechsel zwei Skalenteile in einer ersten Farbe und anschließend drei Skalenteile in einer nächsten Farbe zu gestalten.

Das Trägermaterial sollte dabei beschriftbar und desinfizierbar ausgestaltet sein. Beispielsweise kann es aus einem Kunststoffmaterial bestehen, das zur Erzeugung eines beruhigenden Hintergrundes eingefärbt sein kann. Alternativ ist es möglich, das Trägerelement aus zwei Kunststofflagen und einer dazwischen angeordneten Gewebelage herzustellen.

Hinsichtlich weiterer vorteilhafter Ausgestaltungen wird auf die Unteransprüche sowie die nachfolgende Beschreibung eines Ausführungsbeispiels unter Bezugnahme auf die beiliegenden Zeichnung verwiesen. In der Zeichnung zeigt:
- Figur 1: ein erstes Spangenteil einer Nagelkorrekturspange gemäß der vorliegenden Erfindung,
- Figur 2: das Spangenteil aus Figur 1 an einem Fußnagel befestigt,
- Figur 3: die Darstellung aus Figur 2 mit einem zweiten Spangenteil der erfindungsgemäßen Korrekturspange, das an den Fußnagel gehängt ist,
- Figuren 4 und 5: den verbindungsvorgang der beiden Spangenteile,
- Figur 6: die erfindungsgemäße Nagelkorrekturspange im Endzustand, und
- Figur 7: ein Prüfmittel gemäß der vorliegenden Erfindung in schematischer Darstellung.

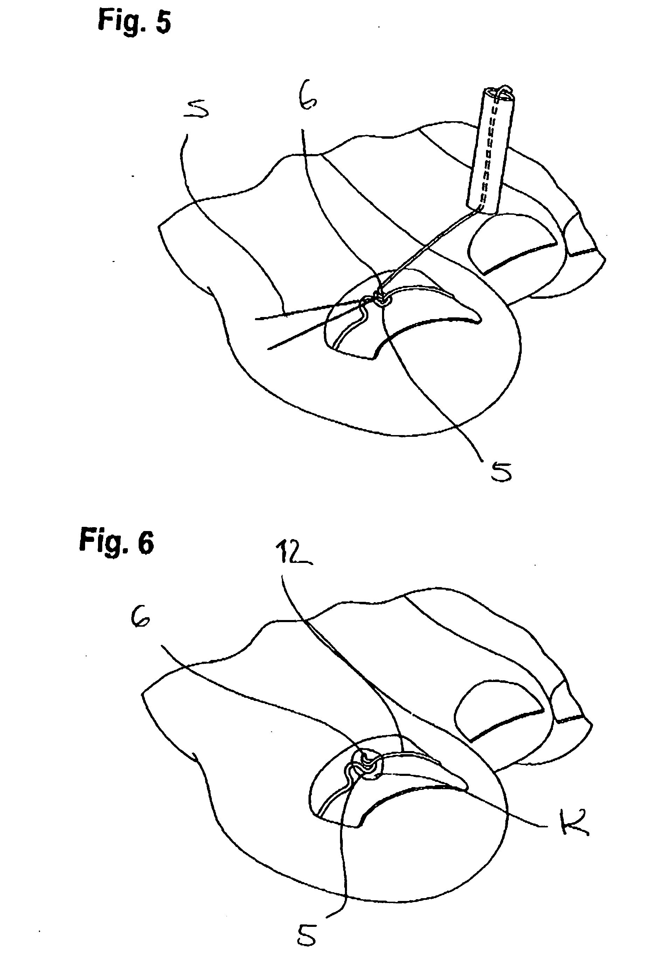

Die Figur 6 zeigt eine Nagelkorrekturspange gemäß der vorliegenden Erfindung, die eingesetzt wird, um zu starke Krümmungen von Nägeln und insbesondere von Fußnägeln N zu behandeln. Die Nagelkorrekturspange besteht aus zwei drahtförmigen Spangenteilen 1, 2, die jeweils an ihrem einen Ende in an sich bekannter Weise einen Endhaken 3, 4 aufweisen, der um eine seitliche Nagelkante gelegt ist, so daß er den Nagel N untergreift. Beabstandet von dem Endhaken 3, 4 weisen die beiden Spangenteile 1, 2 jeweils eine Verbindungsausbiegung 5, 6 auf, über welche die beiden Spangenteile 1, 2 verhakt und auf diese Weise unmittelbar miteinander verbunden sind.

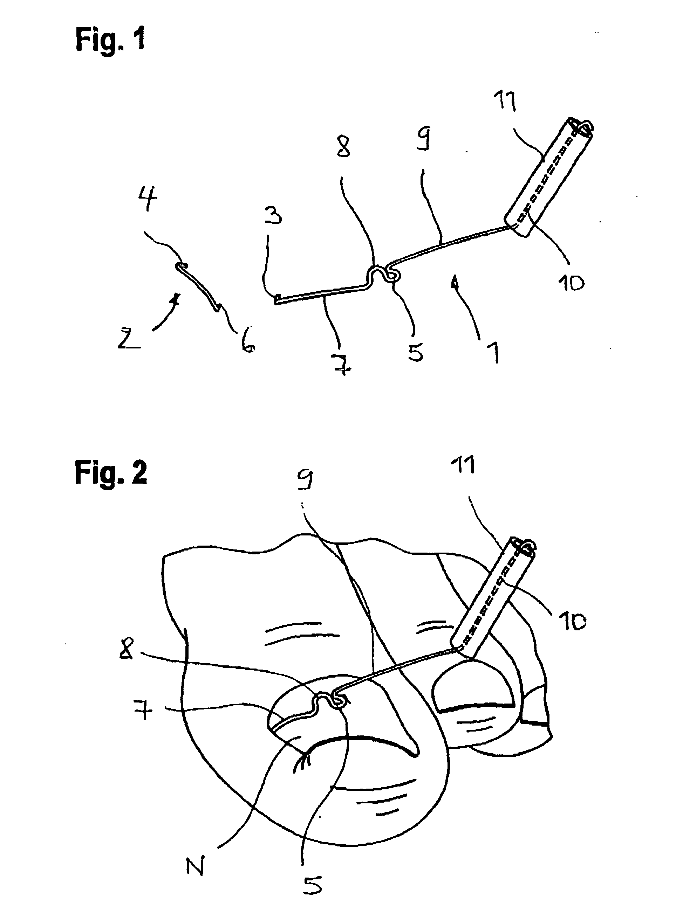

In der Figur 1 sind die beiden Spangenteile 1, 2 einzeln dargestellt. Das erste Spangenteil 1 besitzt - ausgehend von seinem in der Figur 1 linken Ende, das hier bereits mit dem Endhaken 3 versehen ist, - einen ersten, gradlinigen oder leicht gekrümmten Drahtschnitt 7, an den sich eine Q- oder U-förmige Federausbiegung 8 anschließt. Unmittelbar im Anschluß an diese Ω-förmige Federausbiegung 8 folgt die Verbindungsausbiegung 5 des ersten Spangenteils, die hier ebenfalls U-förmig ausgebildet ist. Dabei ist die Ebene der Verbindungsausbiegung 5 quer und insbesondere orthogonal zu der Ebene der Federausbiegung 8 ausgerichtet, so daß, wenn die Federausbiegung 8 auf einem Nagel N flach aufliegt, die Verbindungsausbiegung 5 von dem Nagel N nach oben absteht. Insbesondere können der Endhaken 3 und die Verbindungsausbiegung 5 in einer Ebene liegen.

An die verbindungsausbiegung 5 schließt sich ein etwa geradlinier oder leicht gebogener Führungsabschnitt 9 an gefolgt von einem Drahtabschnitt 10, an dem ein Griffelement 11 befestigt ist.

Das zweite Spangenelement 2 besteht im wesentlichen aus einem leicht gekrümmten Drahtabschnitt 12, der an seinem einen Ende die Verbindungsausbiegung 6 und an seinem anderen Ende den Endhaken 4 aufweist.

In den Figuren 2 bis 6 ist dargestellt, wie die erfindungsgemäße Nagelkorrekturspange bestehend aus den beiden Spangenteilen 1, 2 an dem Nagel N des dicken zehs eines rechten Fußes angebracht wird. Der Therapeut, welcher die Nagelkorrekturspange anbringt, prüft zunächst, ob eine gleichmäßige Korrektur an beiden Nagelrändern erforderlich ist oder der Nagel N sich an einer Seite stärker in die Nagelfalze eindrückt. Bei einer gleichmäßigen Korrektur wird die Federausbiegung 8 etwa auf dem höchsten Punkt des Nagels N, d. h. etwa mittig zwischen den beiden Nagelrändern, positioniert. Wenn an einer Seite eine stärkere Korrektur erforderlich ist, wird die Federausbiegung 8 in Richtung dieses Nagelrandes verlagert, wie dies im dargestellten Ausführungsbeispiel der Fall ist. Anschließend wird der erste Drahtabschnitt 7 des ersten Spangenteils 1 entsprechend der Form des Nagels N gebogen, ggf. in der Länge gekürzt und schließlich der Endhaken 3 an dem freien Ende des Drahtabschnitts 7 angeformt. Dann wird der Endhaken 3 punktgenau unter den Nagelrand eingehakt und auf diese Weise das erste Spangenteil 1 an dem Nagel N angebracht. Der Punkt, an dem die beiden Spangenteile 1, 2 später verbunden werden, wird nun bestimmt und angezeichnet. Wesentlich ist hierbei, daß die Federausbiegung 8 flach auf dem Nagel N zu liegen kommt und ihre Öffnung zu der freien, vorderen Nagelkante weist. Wie die Figur 2 erkennen läßt, steht in diesem Zustand die Verbindungsausbiegung 5 des ersten Spangenteils 1 von dem Nagel N nach oben ab und ragt der Führungsabschnitt 9 in einen spitzen Winkel von der Verbindungsausbiegung 5 nach oben. Das Griffelement 11 steht vom dem Führungsabschnitt 9 nach oben ab, so daß es von dem Therapeuten gut gegriffen werden kann. Die genaue Positionierung kann von dem Therapeuten letztendlich selbst bestimmt werden, indem dieser den Draht entsprechend biegt und kürzt. Schließlich wird das erste Spangenteil 1 in seinem an den Endhaken anschließenden Drahtabschnitt 7 hochgebogen, und zwar zweckmäßigerweise um das Maß der Nageldicke von 1 bis 3 mm, und auf diese weise aktiviert.

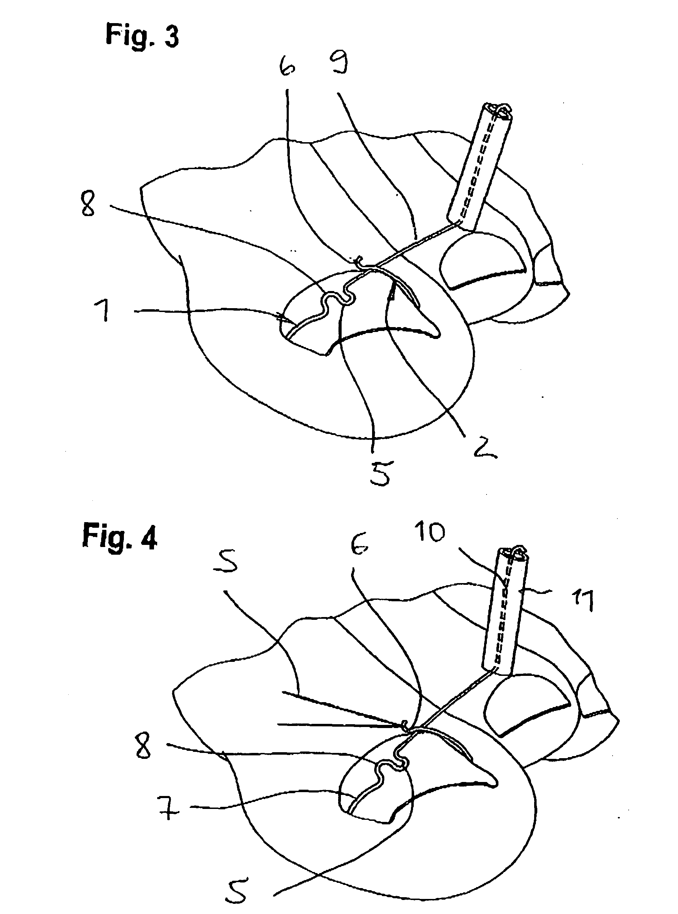

Nach erfolgter Anbringung des ersten Spangenteils 1 wird das zweite Spangenteil 2 entsprechend der Nagelform gebogen, auf Länge anhand der angezeichneten Markierung zugeschnitten und mit dem Endhaken 4, über welchen er später an dem gegenüberliegenden Nagelrand eingehängt wird (siehe Figur 3), und der Verbindungsausbiegung 6 versehen. Anschließend wird in die verbindungsausbiegung 6 des zweiten Spangenteils 2 eine Schlaufe S beispielsweise aus Draht eingehängt, die an einem Griffelement oder einer Fingerschlaufe, welche über einen Finger gestülpt werden kann, befestigt ist. Nun wird die Verbindungsausbiegung 6 des zweiten Spangenteils 2 in Eingriff mit dem Führungsabschnitt 9 des ersten Spangenteils 1 gebracht und mittels der Schlaufe S entlang des Führungsabschnitts 9 in Eingriff mit der Verbindungsausbiegung 5 des ersten Spangenteils 1 gezogen (siehe Figuren 4 und 5).

Dabei wird das erste Spangenteil so gegen den Nagel elastisch nach unten gezogen und am zweiten Spangenteil 2 gespannt gehalten, daß die Spange absichtlich genug Spannkraft hat, um den zu korrigierenden Nagelrand anzuheben. Anschließend werden der Führungsabschnitt 9 und der Haltebereich 10 von dem ersten Spangenteil 1 abgeschnitten. Schließlich wird die Nagelkorrekturspange im Verbindungsbereich der beiden Spangenteile 1, 2 am Nagel N fixiert, beispielsweise mit einem Kunststoff K verklebt, so daß im verbindungsbereiche keine verschiebungen oder Verformungen mehr möglich sind.

Hierbei kann auch das zweite Spangenteil 2 aktiviert werden, indem es vor der Verbindung mit dem ersten Spangenteil 1 vom Nagel N hochgebogen wird.

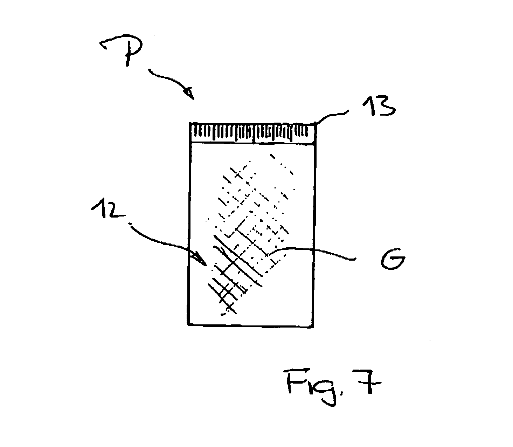

In der Figur 7 ist ein Prüfmittel P zur Feststellung des Erfolgs der Nagelkorrektur dargestellt. Das Prüfmittel P besteht aus einem Trägerelement 12 mit einer rechteckigen Grundform und abgerundeten Ecken, an dessen einem stirnseitigen Endbereich eine Skala 13 - hier eine Milimeterskala - vorgesehen ist. Das Trägerelement 12 wird hier von zwei beschriftbaren und desinfizierbaren transparenten Kunststofflagen und einer dazwischen liegenden Gewebelage G gebildet. Die Gewebelage G dient dazu, den Hintergrund zu beruhigen. Die Skala 13 ist in sich abwechselnden Blöcken von jeweils zwei Skalenabschnitten und anschließend drei Skalenabschnitten unterschiedlich farbig eingeteilt, um das Ablesen zu erleichtern.

## Patentansprüche

1. Nagelkorrekturspange, die quer über einen zu behandelnden Nagel (N) legbar ist und endseitig jeweils einen Endhaken (3,4) zur Anbringung an dem Nagel (N) aufweist, der um eine seitliche Nagelkante legbar ist, so daß er den Nagel (N) untergreift, wobei sie aus zwei drahtförmigen Spangenteilen (1, 2) besteht, die jeweils an ihrem einen Ende einen der Endhaken (3,4) zur Anbringung an dem Nagel (N) und beabstandet von dem Endhaken (3,4) eine Verbindungsausbiegung (5, 6) aufweisen, um die beiden Spangenteile (1, 2) unmittelbar miteinander zu verbinden und wobei sich an die Verbindungsausbiegung (5) von einem der Spangenteile (1) ein Führungsabschnitt (9) für die Verbindungsausbiegung (6) des anderen Spangenteils (2) anschließt.

2. Nagelkorrekturspange nach Anspruch 1, **dadurch gekennzeichnet, daß** ein erstes Spangenteil (1) eine Ω- oder U-förmige Federausbiegung (8) aufweist, die nach der Anbringung an einem Nagel (N) flach auf diesem aufliegt und deren offene Seite zu der freien, vorderen Nagelkante weist.

3. Nagelkorrekturspange nach Anspruch 2, **dadurch gekennzeichnet, daß** die Federausbiegung (8) neben der Verbindungsausbiegung (5) des ersten Spangenteils (1) vorgesehen ist.

4. Nagelkorrekturspange nach Anspruch 3, **dadurch gekennzeichnet, daß** die Verbindungsausbiegung (5) des ersten Spangenteils (1) Ω- oder U-förmig ausgebildet und so ausgerichtet ist, so daß ihr offenes Ende von der freien, vorderen Nagelkante wegweist.

5. Nagelkorrekturspange nach Anspruch 4, **dadurch gekennzeichnet, daß** die Verbindungsausbiegung (5) quer zu der Ebene der Federausbiegung (8) ausgerichtet ist, so daß die Verbindungsausbiegung (5) bei an einem Nagel (N) befestigter Spange von dem Nagel (N) nach oben absteht.

6. Nagelkorrekturspange nach einem der vorherigen Ansprüche, **dadurch gekennzeichnet, daß** sich an den Führungsabschnitt (9) ein Drahtabschnitt (10) anschließt, an dem ein Griffelement (11) vorgesehen ist.

7. Nagelkorrekturspange nach einem der vorherigen Anspruche mit einem Werkzeug zur Anbringung der Nagelkorrekturspange mit einer Schlaufe (S), die an der Verbindungsausbiegung (6) eingehängt wird, um diese über den Führungsabschnitt (9) zu ziehen und in Eingriff mit der Verbindungsausbiegung (5) bringen zu können, wobei die Schlaufe (S) an ein Halteelement in Form eines über ein Finger ziehbaren Rings oder über einen Finger ziehbaren Schlaufe angebracht ist.

## Claims

1. A nail correction clasp which can be placed crosswise over a nail (N) to be treated and has a respective end hook (3,4) on either side for attachment to the nail (N), and which can be placed around a side edge of the nail so that it engages beneath the nail (N), said clasp being made up of two wire-type clasp portions (1, 2) which respectively have at their one end one of the end hooks (3,4) for attachment to the nail (N), and spaced apart from the end hook (3,4) a connection bend (5,6) in order to connect the two clasp portions (1,2) directly to one another, and a guide section (9) for the connection bend (6) of the other clasp portion (2) adjoining the connection bend (5) of one of the clasp portions (1).

2. The nail correction clasp according to Claim 1, **characterised in that** a first clasp portion (1) has a Ω- or U-shaped sprung bend (8) which lies flat on a nail (N) after attachment to the latter, and the open side of which faces towards the free, front edge of the nail.

3. The nail correction clasp according to Claim 2, **characterised in that** the sprung bend (8) is provided next to the connection bend (5) of the first clasp portion (1).

4. The nail correction clasp according to Claim 3, **characterised in that** the connection bend (5) of the first clasp portion (1) is Ω- or U-shaped in design, and is aligned such that its open end faces away from the free, front edge of the nail.

5. The nail correction clasp according to Claim 4, **characterised in that** the connection bend (5) is aligned crosswise to the plane of the sprung bend (8) so that the connection bend (5) projects upwards from the nail (N) when the clasp is attached to a nail (N).

6. The nail correction clasp according to any of the preceding claims, **characterised in that** a wire section (10), on which a grip element (11) is provided, adjoins the guide section (9).

7. The nail correction clasp according to any of the preceding claims with a tool for attaching the nail correction clasp to a nail, with a loop (S) which is hooked onto the connection bend (6) in order to pull the latter over the guide section (9) and to be able to engage it with the connection bend (5), the loop (S) being attached to a holding element in the form of a ring which can be pulled over a finger or a loop which can be pulled over a finger.

## Revendications

1. Agrafe de correction d'ongles, qui est déposée transversalement sur l'ongle à traiter (N) et présente à chaque extrémité un crochet (3,4) pour un emplacement à l'ongle (N) et qui, placé latéralement sous le bord de l'ongle (N), accroche dans ce dernier, l'agrafe étant constituée de deux éléments (1,2) sous forme de fil présentant chacun à l'une de ces extrémités un crochet (3,4) pour la mise en place à l'ongle (N) et à un certain écart du crochet (3,4), un déversement de liaison (5,6), permettant une liaison direct des deux éléments (1,2) d'agrafe, et en ce faisant, un tronçon de guidage (9) est attaché au déversement de liaison (5) de l'un des deux éléments d'agrafe (1), pour le déversement de liaison (6) de l'autre élément d'agrafe (2).

2. Agrafe de correction d'ongles selon revendication 1 **caractérisée en ce que**, un premier élément d'agrafe (1) présente un déversement de ressort (8) sous forme d'Ω ou bien U, et qui après l'emplacement à un ongle (N), repose à plat sur ce dernier et montre avec son coté ouvert vers le bord libre de l'ongle.

3. Agrafe de correction d'ongles selon revendication 2 **caractérisée en ce que**, le déversement de ressort (8) est prévu à coté du déversement de liaison (5) du premier élément (1) de l'agrafe.

4. Agrafe de correction d'ongles selon revendication 3 **caractérisée en ce que**, le déversement de liaison (5) du premier élément (1) de l'agrafe est formé sous forme d'Ω ou U et placé de telle façon que, son extrémité ouverte est détournée de l'avant bord de l'ongle.

5. Agrafe de correction d'ongles selon revendication 4 **caractérisée en ce que**, le déversement de liaison (5) est ajusté transversalement au plan du déversement de ressort (8), de telle façon que, le déversement de liaison (5), s'élève de l'ongle (N) vers le haut, après l'emplacement de l'agrafe sur l'ongle (N).

6. Agrafe de correction d'ongles selon l'une des revendications préalables **caractérisée en ce que**, une poignée (11) est prévue à un tronçon de fil (10) qui est attaché au tronçon de guidage (9).

7. Agrafe de correction d'ongles selon l'une des revendications préalables **caractérisée en ce que**, un passant S est attaché au déversement de liaison (6) à fin de permettre le raccordement de ce dernier en le tirant au long du tronçon de guidage (9), avec le déversement de liaison (5), ce faisant, le passant (S) est fixé sur un appui en forme d'une bague de doigt ou d'une sangle.
